# EUROPÄISCHE PATENTSCHRIFT

(11) **EP 1 037 884 B1**
(45) Veröffentlichungstag und Bekanntmachung des Hinweises auf die Patenterteilung: **13.03.2002**
(21) Anmeldenummer: 98962407.7
(22) Anmeldetag: 02.12.1998
(51) Int. Cl.: C07D 307/32

(54) **VERFAHREN ZUR ABTRENNUNG VON VERUNREINIGUNGEN AUS 3-(2'-ACETOXYETHYL)-DIHYDRO-2(3H)FURANON**
METHOD FOR SEPARATING CONTAMINANTS FROM 3-(2'-ACETOXY-ETHYL-DIHYDRO-2(3H)FURANONE
PROCEDE POUR LA SEPARATION D'IMPURETES DANS DE LA 3-(2'-ACETOXYETHYL)-DIHYDRO-2 (3H) FURANONE

(30) Priorität: 08.12.1997 DE 19754302
(43) Veröffentlichungstag der Anmeldung: 27.09.2000
(73) Patentinhaber: BASF AKTIENGESELLSCHAFT, 67056 Ludwigshafen (DE)
(72) Erfinder: MAYWALD, Volker, D-67071 Ludwigshafen (DE); GÖTZ, Norbert, D-67547 Worms (DE); KÜKENHÖHNER, Thomas, D-67459 Böhl-Iggelheim (DE); BORCHERS, Dirk, D-67134 Birkenheide (DE); KÖNIG, Hartmann, D-69115 Heidelberg (DE); HARTMANN, Horst, D-67459 Böhl-Iggelheim (DE); WAGNER, Rupert, D-67227 Frankenthal (DE)
(86) Internationale Anmeldenummer: EP9807832
(87) Internationale Veröffentlichungsnummer: WO9929680

(56) Entgegenhaltungen:
- EP-A- 0 246 581
- EP-A- 0 584 631
- EP-A- 0 588 224
- US-A- 2 443 827

## Beschreibung

Die vorliegende Erfindung betrifft ein Verfahren zur Abtrennung unerwünschter Verunreinigungen aus 3-(2'-Acetoxyethyl)-dihydro-2(3H)furanon der Formel I

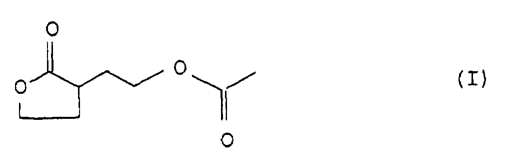

3-(2'-Acetoxyethyl)-dihydro-2(3H)furanon ist ein Ausgangsprodukt zur Herstellung von Tetrahydropyran-4-carbonsäure-methylester, welcher seinerseits ein Vorprodukt bei der Herstellung von Pflanzenschutzmitteln ist.

3-(2'-Acetoxyethyl)-dihydro-2(3H)furanon wird z.B. nach an sich bekannten und z.B. in der US-A 5,350,863 beschriebenen Verfahren ausgehend von Acetessigsäuremethylester und Ethylenoxid hergestellt. Eine andere Verfahrensvariante, nach der 3-(2'-Acetoxyethyl)-dihydro-2(3H)furanon erhältlich ist, ist in Dokl.Akad. NAuk SSSR 27, 956-959 (1940) und der US-A 5,283,326 beschrieben.

Nach beiden Varianten entsteht das gewünschte 3-(2'-Acetoxyethyl)-dihydro-2(3H)furanon im Gemisch mit einer Reihe unerwünschter Nebenkomponenten, insbesondere dem isomeren Dihydro-3-(2-methyl-1,3-dioxolan-2-yl)-2(3H)-furanon II

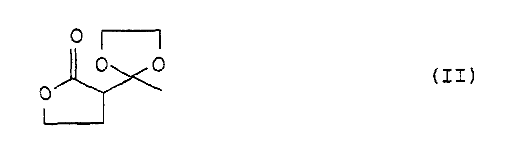

Wird das derart verunreinigte I in einer kontinuierlichen Gasphasenreaktion mit Methanol in Gegenwart saurer Katalysatoren zu Tetrahydropyran-4-carbonsäuremethylester umgesetzt, so werden deutlich niedrigere Ausbeuten und kürzere Katalysatorstandzeiten erhalten als bei Einsatz eines hochreinen Produkts ohne die Nebenkomponenten. Dies ist wahrscheinlich darauf zurückzuführen, daß die meisten Nebenkomponenten empfindliche Acetalgruppen enthalten, die sich an der Katalysatoroberfläche zu oligomeren und polymeren Produkten zersetzen.

Die Abtrennung vor allem von II aus Mischungen, die I und II enthalten, ist destillativ aufgrund der sehr nahe beieinanderliegenden Siedepunkte nur schwer und kostenaufwendig zu realisieren.

Es bestand daher die Aufgabe, ein Verfahren zur Verfügung zu stellen, nach welchem unerwünschte Nebenkomponenten, insbesondere II aus Mischungen mit I entfernt werden können, ohne daß I hierbei selbst verändert wird.

Diese Aufgabe wurde erfindungsgemäß gelöst durch ein Verfahren zur Abtrennung von Verunreinigungen aus 3-(2'-Acetoxyethyl)-dihydro-2(3H)furanon (I), wobei das die unerwünschten Verunreinigungen enthaltende 3-(2'-Acetoxyethyl)-dihydro-2(3H)furanon zunächst in an sich bekannter Weise durch Acetylierung von 3-(2'-Hydroxyethyl)-dihydro-2-(3H)furanon hergestellt, anschließend mit starken Mineralsäuren behandelt und schließlich die Zersetzungsprodukte der unerwünschten Verunreinigungen von I abgetrennt werden.

Überraschenderweise wird das gewünschte I bei der Behandlung mit Mineralsäuren nicht abgebaut und kann daher in guter Ausbeute mit hoher Reinheit gewonnen werden.

Bevorzugte Ausführungsformen des erfindungsgemäßen Verfahrens sind den Unteransprüchen und der nachfolgenden Beschreibung zu entnehmen.

Ausgangspunkt des erfindungsgemäßen Verfahrens ist das nach bekannten und in der Einleitung beschriebenen Verfahren erhältliche Gemisch aus 3-(2'-Hydroxyethyl)-dihydro-2(3H)furanon und unerwünschten Verunreinigungen.

Dieses Gemisch wird zunächst weitgehend vollständig acetyliert. Als Acetylierungsmittel verwendet man vorzugsweise Acetanhydrid oder Essigsäure selbst; grundsätzlich sind jedoch alle dem Fachmann für entsprechende Acetylierungen bekannten Acetylierungsmittel geeignet. Die Acetylierung wird in der Regel bei Temperaturen im Bereich von 40 °C bis 200 °C, vorzugsweise von 60 bis 140 °C über einen Zeitraum von 0,5 bis 10, vorzugsweise 0,8 bis 5 und insbesondere 1 bis 3 h durchgeführt.

Um eine vollständige Acetylierung sicherzustellen, wird das Acetylierungsmittel in der Regel in einem molaren Überschuß von 5. bis 50 %, vorzugsweise von 5 bis 20 %, bezogen auf das im Gemisch vorliegende 3-(2'-Hydroxyethyl)-dihydro-2(3H)furanon eingesetzt.

Im nachfolgenden Schritt wird das bei der Acetylierung entstandene Produkt mit starken Mineralsäuren behandelt. Bevorzugte Mineralsäuren sind Salzsäure, Salpetersäure und besonders bevorzugt Schwefelsäure. Diese wird bevorzugt mindestens 80%ig und besonders in Form von konzentrierter Schwefelsäure eingesetzt.

Die Menge an eingesetzter Schwefelsäure kann dabei in weiten Bereichen variiert werden; es hat sich in manchen Fällen als vorteilhaft herausgestellt, wenn das molare Verhältnis von starker Mineralsäure, bezogen auf die in der vorhergehenden Stufe eingesetzte Menge an Acetylierungsmittel im Bereich von 1: 20 bis 1:3, insbesondere von 1:15 bis 1:7 und insbesondere 1:8 bis 1:12 beträgt.

Die Temperatur bei der Behandlung mit der Mineralsäure liegt im allgemeinen im Bereich von 10 bis 80, vorzugsweise von 20 bis 60 und insbesondere von 30 bis 50 °C. Die Dauer der Behandlung liegt im allgemeinen im Bereich von 0,3 bis 10, insbesondere von 1 bis 5 h, kann aber grundsätzlich in weiten Grenzen variiert werden.

Nach der Behandlung mit starken Mineralsäuren lassen sich die Zersetzungsprodukte der unerwünschten Verunreinigungen, insbesondere des Dihydro-3-(2-methyl-1,3-dioxolan-2-yl)-2(3H)-furanon II einfach abtrennen.

Nach einer bevorzugten Variante wird durch Zugabe von Base, vorzugsweise wäßrigem Alkalihydroxid, zunächst die Mineralsäure neutralisiert und ggf. anschließend die bei der Acetylierung als Koppelprodukt entstandene Essigsäure abdestilliert. Anschließend wird durch Zugabe eines geeigneten organischen Lösungsmittels und Wasser eine Phasentrennung vorgenommen und anschließend die organische Phase extrahiert. Als organische Lösungsmittel eignen sich insbesondere aromatische Kohlenwassertoffe und besonders alkylierte Benzolderivate wie Xylol oder Toluol. Nach Vereinigung der organischen Extrakte und Entfernung des Lösungsmittels kann der Rückstand im Vakuum rektifiziert werden, wobei man in guter Ausbeute und hoher Reinheit das gewünschte Produkt erhält, welches anschließend weiter verarbeitet werden kann. Der Reinheitsgrad von I kann dabei noch erhöht werden, indem man mehrere Rektifikationsstufen hintereinander ausführt. Die Bedingungen für die Rektifikation sind dem Fachmann an sich bekannt und in der Literatur beschrieben, so daß sich hier weitere Angaben erübrigen.

Die Reinheit von I nach Durchführung des erfindungsgemäßen Verfahrens liegt in der Regel bei mindestens 98, vorzugsweise bei mindestens 98,5 und besonders bevorzugt bei mindestens 99 Gew%.

Das nach dem erfindungsgemäßen Verfahren erhältliche I läßt sich in an sich bekannter Weise in hoher Ausbeute und mit guten Katalysatorstandzeiten zu Tetrahydropyran-4-carbonsäuremethylester umsetzen. Dieses ist ein wichtiges Vorprodukt bei der Herstellung von Pflanzenschutzmitteln.

### Beispiel 1 (Vergleich)

In einem 3,5 1 Druckbehälter wurde eine Mischung aus 1009 g (8,7 mol) Acecessigsäuremethylester, 1218 ml (969 g) Methanol und 110 g (0,61 mol) einer 30%igen Natriummethylat-Lösung in Methanol vorgelegt und anschließend 765,6 g (17,4 mol) Ethylenoxid bei 60°C unter Rühren innerhalb von 8 Stunden zugepumpt. Danach wurde 24 h bei 60°C nachgerührt. Der Reaktionsaustrag wurde mehrfach mit Stickstoff gespült und anschließend in eine Rührapparatur mit aufgesetzter Kolonne überführt. Danach wurde der Katalysator durch Zugabe von 29,6 g (0,3 mol) Schwefelsäure (96%) neutralisiert. Die niedrig siedenden Anteile, im wesentlichen Methanol, Methylacetat und Methylglykol, wurden bis zu einer Sumpftemperatur von 100°C bei 10 mbar (1013 Pa) abdestilliert. Zum Destillationsrückstand wurden bei 100°C innerhalb von einer Stunde unter Rühren 806 g (7,9 mol) Acetanhydrid gegeben und 2 h bei 100°C nachgerührt. Anschließend wurde das überschüssige Acetanhydrid sowie die als Koppelprodukt bei der Acetylierung entstandene Essigsäure abdestilliert und der Rohaustrag abgekühlt. Nach Zugabe von 726 g Toluol und 300 g Wasser wurden die Phasen getrennt und die wäßrige Phase zweimal mit je 200 ml Toluol extrahiert. Die organischen Extrakte wurden vereinigt und das Solvens am Rotationsverdampfer im Vakuum abgezogen. Der Rückstand wurde im Vakuum bei 10 mbar diskontinuierlich rektifiziert (Sdp. 161 °C/10 mbar).
Man erhielt 1077 g (72%) 3-(2'-Acetoxyethyl)-dihydro-2(3H)furanon (berechnet 100%).

| Zusammensetzung: | |
|---|---|
| 3-(2'-Acetoxyethyl)-dihydro-2(3H)furanon | 95,0% |
| Dihydro-3-(2-methyl-1,3-dioxolan-2-yl)-2(3H)-furanon | 3,5%. |
| 3-(2'-Hydroxyethyl)-dihydro-2(3H)furanon | 0,50%, |
| Sonstige | 1,0% |

### Beispiel 2 ((einfache diskontinuierliche Rektifikation)

In einem 3,5 1 Druckbehälter wurde eine Mischung aus 1009 g (8,7 mol) Acetessigsäuremethylester, 1218 ml (969 g) Methanol und 110 g (0,61 mol) einer 30 %igen Natriummethylat-Lösung in Methanoi vorgelegt und anschließend 765,6 g (17,4 mol) Ethylenoxid bei 60°C unter Rühren innerhalb von 8 Stunden zugepumpt. Danach wurde 24 h bei 60°C nachgerührt. Der Reaktionsaustrag wurde mehrfach mit Stickstoff gespült und anschließend in eine Rührapparatur mit aufgesetzter Kolonne überführt. Danach wurde der Katalysator durch Zugabe von 29,6 g (0,3 mol) Schwefelsäure (96%) neutralisiert. Die niedrig siedenden Anteile, im wesentlichen Methanol, Methylacetat und Methylglykol, wurden bis zu einer Sumpftemperatur von 100°C bei 10 mbar abdestilliert. Zum Destillationsrückstand wurden bei 100°C innerhalb von einer Stunde unter Rühren 806 g (7,9 mol) Acetanhydrid gegeben und 2 h bei 100°C nachgerührt. Zur Zersetzung des Dihydro-3-(2-methyl-1,3-dioxolan-2-yl)-2(3H)-furanons wurde das Reaktionsgemisch auf 40°C abgekühlt, 72,5 g (0,73 mol) Schwefelsäure (96%) zugegeben und zwei Stunden bei dieser Temperatur nachgerührt. Zur Neutralisation der Schwefelsäure wurden anschließend 127,5 g (1,59 mol) 50%ige NaOH bei 40-45°C zugegeben. Die bei der Acetylierung als Koppelprodukt entstandene Essigsäure wurde abdestilliert und der Rohaustrag abgekühlt. Nach Zugabe von 726 g Toluol und 850 g Wasser wurden die Phasen getrennt und die wäßrige Phase zweimal mit je 200 ml Toluol extrahiert. Die organischen Extrakte wurden vereinigt und das Solvens am Rotationsverdampfer im Vakuum abgezogen. Der Rückstand wurde im Vakuum bei 10 mbar diskontinuierlich rektifiziert (Sdp. 161°C/10 mbar). Man erhielt 1056 g (71%) 3-(2'-Acetoxyethyl)-dihydro-2(3H)furanon (berechnet 100%).

| Zusammensetzung: | |
|---|---|
| 3-(2'-Acetoxyethyl)-dihydro-2(3H)furanon | 98,5% |
| Dihydro-3-(2-methyl-1,3-dioxolan-2-yl)-2(3H)-furanon | 0,3% |
| 3-(2'-Hydroxyethyl)-dihydro-2(3H)furanon | 0,3% |
| Sonstige | 0,9% |

### Beispiel 3 (dreifache kont. Rektifikation)

In einem 1 m³ Druckbehälter wurde eine Mischung aus 348 kg (3,0 kmol) Acetessigsäuremethylester, 420,1 l (334,2 kg) Methanol und 37,9 kg (210,3 mol) einer 30%igen Natriummethylat-Lösung in Methanol vorgelegt und anschließend 264,1 kg (6,0 kmol) Ethylenoxid bei 60°C unter Rühren innerhalb von 8 Stunden zugepumpt. Danach wurde 24 h bei 60°C nachgerährt. Der Reaktionsaustrag wurde mehrfach mit Stickstoff gespült und anschließend in eine 1 m3 Ruhrapparatur mit aufgesetzter Kolonne überführt. Danach wurde der Katalysator durch Zugabe von 10,2 kg (103,5 mol) Schwefelsäure (96%) neutralisiert. Die niedrig siedenden Anteile, im wesentlichen Methanol, Methylacetat und Methylglykol, wurden bis zu einer Sumpftemperatur von 100°C bei 10 mbar (1013 Pa) abdestilliert. Zum Destillationsrückstand wurden bei 100°C innerhalb von einer Stunde unter Rühren 278 kg (2,7 kmol) Acetanhydrid gegeben und 2 h bei 100°C nachgerührt. Zur Zersetzung des Dihydro-3-(2-methyl-1,3-dioxolan-2-yl)-2(3H)-furanons wurde das Reaktionsgemisch auf 40°C abgekühlt, 25,0 kg (251,8 mol) Schwefelsäure (96%) zugegeben und zwei Stunden bei dieser Temperatur nachgerührt. Zur Neutralisation der Schwefelsäure wurden anschließend 439,7 kg (548 mol) 50%ige NaOH bei 40-45°C zugegeben. Die bei der Acetylierung als Koppelprodukt entstandene Essigsäure wurde abdestilliert und der Rohaustrag abgekühlt. Nach Zugabe von 250,4 kg Toluol und 293,2 kg Wasser wurden die Phasen getrennt und die wäßrige Phase zweimal mit je 100 kg Toluol extrahiert. Die organischen Extrakte wurden vereinigt und weiter aufgearbeitet: Man erhielt 375,3 kg (73%) 3-(2'-Acetoxyethyl)dihydro-2(3H)furanon (berechnet 100%).

| Zusammensetzung: | |
|---|---|
| 3-(2'-Acetoxyethyl)-dihydro-2(3H)furanon | 99,5% |
| Dihydro-3-(2-methyl-1,3-dioxolan-2-yl)-2(3H)-furanon | <0,1% |
| 3-(2'-Hydroxyethyl)-dihydro-2(3H)furanon | 0,3% |
| Sonstige | 0,1% |

Um die Auswirkungen des Reinheitsgrades von I auf die nachfolgende Umsetzung zu untersuchen, wurden die nach den Beispielen 1 bis 3 erhaltenen Produkte zu Tetrahydropyran-4-carbonsäuremethylester gemäß folgender allgemeiner Gleichung umgesetzt:

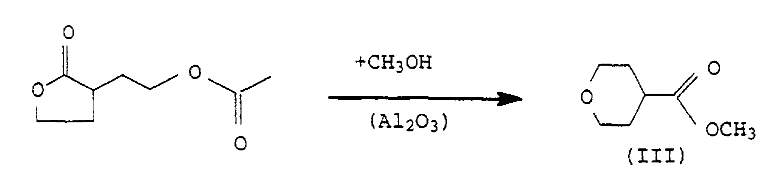

Pro Stunde wurde eine Lösung bestehend aus 172 g 3-(2'-Acetoxyethyl)-dihydro-2(3H)furanon und 192 g Methanol verdampft und in einem Rohrreaktor bei 250°C über 2000 g eines γ-Aluminiumoxid-Katalysators (Pural SB™, zu 2 mm Strängen verformt, bei 120°C/16 h getrocknet und 520°C/3 h calciniert) geleitet. Der gasformige Reaktionsaustrag wurde kondensiert und der entstandene Tetrahydropyran-4-carbonsäureester durch diskontinuierliche Destillation gereinigt (Sdp. 117°C/30 mbar).

Die Ergebnisse der Umsetzungen von 3-(2'-Acetoxyethyl)-dihydro-2(3H)furanon I mit verschiedenen Anteilen Dihydro-3-(2-methyl-1,3-dioxolan-2-yl)-2(3H)-furanon II sind in Tabelle 1 zusammengefaßt:

**Tabelle 1:**

| Anteil II in % | Umsatz (%) | Ausbeute an III, zu Beginn | Ausbeute an III, nach 60 h | Ausbeute an III, nach 1200h | Ausbeute an III, nach 1440h |
|---|---|---|---|---|---|
| 3,5(Bsp.1) | 100 | 55% | 39% | - | - |
| 0,3(Bsp.2) | 100 | 68% | 68% | 68% | 57% |
| <0,1(Bsp.3) | 100 | 68% | 68% | 68% | 68% |

Die Ergebnisse der vorstehenden Tabelle zeigen den starken Einfluß des Gehalts an Verunreinigungen, insbesondere II, auf die weitere Verarbeitung von I.

## Patentansprüche

1. Verfahren zur Abtrennung von Verunreinigungen aus 3-(2'-Acetoxyethyl)-dihydro-2(3H)furanon (I), **dadurch gekennzeichnet, daß** das die unerwünschten Verunreinigungen enthaltende 3-(2'-Acetoxyethyl)-dihydro-2(3H)furanon zunächst in an sich bekannter Weise durch Acetylierung von 3-(2'-Hydroxyethyl)-dihydro-2-(3H)furanon hergestellt und anschließend mit starken Mineralsäuren behandelt wird und schließlich die Zersetzungsprodukte der unerwünschten Verunreinigungen von I abgetrennt werden.

2. Verfahren nach Anspruch 1, **dadurch gekennzeichnet, daß** man als Acetylierungsmittel Acetanhydrid oder Essigsäure einsetzt.

3. Verfahren nach einem der Ansprüche 1 oder 2, **dadurch gekennzeichnet, daß** man als starke Mineralsäure Schwefelsäure verwendet.

4. Verfahren nach einem der Ansprüche 1 bis 3, **dadurch gekennzeichnet, daß** man die Behandlung mit Schwefelsäure bei Temperaturen von 20 bis 70 °C durchführt.

5. Verfahren nach einem der Ansprüche 1 bis 4, **dadurch gekennzeichnet, daß** man die Zersetzungsprodukte durch Phasentrennung,anschließende Extraktion und Destillation bzw. Rektifikation abtrennt.

6. Verfahren nach einem der Ansprüche 5, **dadurch gekennzeichnet, daß** man eine mehrstufige Destillation bzw. Rektifikation durchführt.

## Claims

1. A process for removing impurities from 3-(2'-acetoxyethyl)-dihydro-2(3H)-furanone (I), which comprises initially preparing the 3-(2'-acetoxyethyl)dihydro-2(3H)-furanone containing the undesirable impurities by acetylating 3-(2'-hydroxyethyl)dihydro-2(3H)-furanone in a manner known per se and subsequently treating it with strong mineral acids and finally removing the decomposition products of the undesirable impurities from I.

2. A process as claimed in claim 1, wherein the acetylating agent used is acetic anhydride or acetic acid.

3. A process as claimed in any of claims 1 or 2, wherein the strong mineral acid used is sulfuric acid.

4. A process as claimed in any of claims 1 to 3, wherein the treatment with sulfuric acid is carried out at temperatures of from 20 to 70°C.

5. A process as claimed in any of claims 1 to 4, wherein the decomposition products are removed by phase separation, subsequent extraction and distillation or rectification.

6. A process as claimed in any of claims [lacuna] 5, wherein a multi-step distillation or rectification is carried out.

## Revendications

1. Procédé pour séparer les impuretés contenues dans une 3-(2'-acétoxyéthyl)-dihydro-2(3H)furannone (I), **caractérisé par le fait que** l'on prépare d'abord la 3-(2'-acétoxyéthyl)dihydro-2(3H)furannone contenant les impuretés gênantes, de manière connue en soi, par acétylation de la 3-(2'-hydroxyéthyl)dihydro-2(3H)furannone puis on la traite par des acides minéraux forts et finalement, on sépare les produits de décomposition des impuretés gênantes de I.

2. Procédé selon la revendication 1, **caractérisé par le fait que** l'on utilise en tant qu'agent acétylant l'anhydride acétique ou l'acide acétique.

3. Procédé selon l'une des revendications 1 ou 2, **caractérisé par le fait que** l'on utilise en tant qu'acide minéral fort l'acide sulfurique.

4. Procédé selon l'une des revendications 1 à 3, **caractérisé par le fait que** l'on procède au traitement par l'acide sulfurique à des températures de 20 à 70°C.

5. Procédé selon l'une des revendications 1 à 4, **caractérisé par le fait que** l'on sépare les produits de décomposition par séparation de phases suivie d'une extraction et d'une distillation ou d'une rectification.

6. Procédé selon l'une des revendications 1 à 5, **caractérisé par le fait que** l'on procède à une distillation ou rectification en plusieurs stades opératoires.
